# EUROPÄISCHE PATENTSCHRIFT

(11) **EP 2 754 406 B1**
(45) Veröffentlichungstag und Bekanntmachung des Hinweises auf die Patenterteilung: **26.06.2019**
(21) Anmeldenummer: 13151104.0
(22) Anmeldetag: 14.01.2013
(51) Int. Cl.: A61C 1/14, A61C 1/08, A61C 1/07, A61C 1/12

(54) **Medizinisches, insbesondere dentales, Handstück**
Medical, in particular dental handpiece
Pièce à main médicale, en particulier dentaire

(43) Veröffentlichungstag der Anmeldung: 16.07.2014
(73) Patentinhaber: W & H Dentalwerk Bürmoos GmbH, 5111 Bürmoos (AT)
(72) Erfinder: Schwarzbraun, Josef, 5112 Lamprechtshausen (AT)
(74) Vertreter: Benda, Ralf

(56) Entgegenhaltungen:
- EP-A1- 1 627 611
- EP-A2- 0 191 574
- EP-A2- 0 360 161
- EP-A2- 0 577 981

## Beschreibung

Die vorliegende Erfindung betrifft ein medizinisches, insbesondere dentales, Handstück mit einer Werkzeughaltehülse zum Halten eine Behandlungswerkzeugs, wobei die Werkzeughaltehülse eine Mittelachse aufweist, um welche die Werkzeughaltehülse drehbar ist, und wobei die Werkzeughaltehülse durch ein Antriebswelle des Handstücks in eine Hubbewegung versetzbar ist.

Ein derartiges medizinisches, insbesondere dentales, Handstück ist aus der Patentanmeldung EP 1 627 611 A1 bekannt. Das Handstück weist des Weiteren eine Stellvorrichtung auf, die wahlweise eine freie Drehbarkeit der Werkzeughaltehülse und eines darin angeordneten Werkzeugs zulässt oder die Drehposition der Werkzeughaltehülse und des darin angeordneten Werkzeugs fixiert. Zum Betätigen der Stellvorrichtung ist ein Betätigungselement in Form eines länglichen Schiebers vorgesehen, der sich entlang des Griffteils des Handstücks erstreckt.

Die Patentanmeldung EP 0 557 981 A2 offenbart ein medizinisches, insbesondere dentales, Handstück mit einer Werkzeughaltehülse zum Halten eine Behandlungswerkzeugs, wobei die Werkzeughaltehülse eine Mittelachse aufweist, um welche die Werkzeughaltehülse drehbar ist, und wobei die Werkzeughaltehülse durch ein Antriebswelle des Handstücks in eine Hubbewegung versetzbar ist. Eine im Handstückkopf vorgesehene Verzahnung erlaubt wahlweise eine freie Drehbarkeit oder fixe Positionierung der Werkzeughaltehülse und eines darin angeordneten Werkzeugs.

Die Patentanmeldung EP 0 360 161 A2 offenbart ein Handstück mit einer in eine Hubbewegung versetzbaren Werkzeughaltehülse zur Aufnahme eines Werkzeugs. Das Werkzeug ist durch eine Kugelspannvorrichtung mit einem relativ zur Werkzeughaltehülse drehbaren Kupplungsring in der Werkzeughaltehülse befestigbar.

Die Patentanmeldung EP 0 191 574 A2 zeigt ebenfalls ein Handstück mit einer in eine Hubbewegung versetzbaren Werkzeughaltehülse zur Aufnahme eines Werkzeugs und einer Kugelspannvorrichtung zum Befestigen des Werkzeugs in der Werkzeughaltehülse.

Der vorliegenden Erfindung liegt die Aufgabe zugrunde, ein medizinisches, insbesondere dentales, Handstück mit einer drehbaren und in eine Hubbewegung versetzbaren Werkzeughaltehülse zu schaffen, welches eine alternative Stellvorrichtung aufweist, deren Betätigungselement sich insbesondere nicht entlang des Griffteils des Handstücks erstreckt.

Diese Aufgabe wird durch ein medizinisches, insbesondere dentales, Handstück gemäß Anspruch 1 gelöst.

Durch das im Wesentlichen parallel zur Mittelachse der Werkzeughaltehülse bewegbare Stellelement ist es möglich, das Betätigungselement zum Betätigen der Stellvorrichtung in axialer Richtung (bezogen auf die Mittelachse der Werkzeughaltehülse) zur Werkzeughaltehülse vorzusehen, insbesondere das Betätigungselement axial angrenzend an die Werkzeughaltehülse oder konzentrisch um die Mittelachse vorzusehen. Bevorzugt ist das Betätigungselement an einem Kopfabschnitt des Handstücks, in welchem die Werkzeughaltehülse angeordnet ist, vorgesehen. Das Betätigungselement erstreckt sich somit nicht mehr, wie aus dem Stand der Technik bekannt, entlang des Griffteils des Handstücks, wodurch das Handstück für den Anwender besser zu halten ist und wodurch am Vorderende des Griffteils, das der Behandlungsstelle besonders nahe ist, keine zusätzlichen Strukturen vorhanden sind, an denen Verschmutzungen, zum Beispiel Behandlungsreste wie Blut oder Gewebe, hängen bleiben können.

Die Werkzeughaltehülse ist vorzugsweise als hohle, rohrförmige oder längliche Hülse ausgebildet. Die Werkzeughaltehülse ist zur lösbaren, vorzugsweise reibschlüssigen und / oder formschlüssigen, Verbindung mit einem Werkzeug ausgebildet. Das Werkzeug umfasst insbesondere eine Werkzeug zur Präparation von Zähnen, zum Beispiel ein feilenartiges Werkzeug, ein Werkzeug mit einem flächigen, abrasiven Arbeitsabschnitt, eine dentale Feile, ein Werkzeug mit einem Sägeblatt oder ein Werkzeug mit einem sägeblattähnlichen Arbeitsabschnitt, insbesondere zum Abtragen von Zahnschmelz.

Bevorzugt sind an der Außenfläche oder am Außenmantel der Werkzeughaltehülse unterschiedliche geometrische Strukturen vorgesehen, zum Beispiel Teile eines Exzentergetriebes, welches die Werkzeughaltehülse in eine Hubbewegung versetzt, insbesondere ein Exzenterstift oder eine Nut oder Ringnut zur Aufnahme eines Exzenterstifts. An der Außenfläche der Werkzeughaltehülse, insbesondere in dem Kontaktbereich mit dem Fixierelement, ist zumindest eine, vorzugsweise konkav geformte, Ausnehmung zur Aufnahme zumindest eines Teils des Fixierelements vorgesehen. Jede Ausnehmung definiert eine festgelegte oder vorbestimmte Drehposition, in welcher die Werkzeughaltehülse fixierbar ist, wenn das Fixierelement in die Ausnehmung eingreift. Vorzugsweise ist die zumindest eine Ausnehmung als längliche Rille oder Nut ausgebildet, die sich entlang der Außenfläche der Werkzeughaltehülse, insbesondere im Wesentlichen parallel zur Mittelachse der Außenhülse, erstrecken, um eine leichtgängige Hubbewegung zu ermöglichen. Die (bezogen auf die Mittelachse der Werkzeughaltehülse) axiale Länge der Rille oder Nut ist zum Beispiel zumindest gleich der axialen Höhe der Nut oder Ringnut zur Aufnahme des Exzenterstiftes des Exzentergetriebes, vorzugsweise größer als die axiale Höhe der Nut oder Ringnut. Alternativ ist die Außenfläche oder der Außenmantel der Werkzeughaltehülse in dem Kontaktbereich mit dem Fixierelement im Wesentlichen glatt oder ohne Ausnehmung zur Aufnahme eines Fixierelements ausgebildet, wodurch die Werkzeughaltehülse in jeder beliebigen Drehposition oder in jedem beliebigen Drehwinkel durch das Fixierelement fixierbar ist.

Gemäß einem Ausführungsbeispiel umfasst das Fixierelement zumindest ein relativ zur Mittelachse der Werkzeughaltehülse im Wesentlichen radial bewegbares, vorzugsweise kugelförmiges oder ein balliges Ende aufweisendes, Formelement. Das Fixierelement ist somit insbesondere derart im Handstück angeordnet, dass es im Wesentlichen radial zur Werkzeughaltehülse bewegbar und von der Werkzeughaltehülse weg bewegbar ist. Besonders bevorzugt ist das Fixierelement als Kugel ausgebildet. Alternativ ist das Formelement zum Beispiel als Zylinder oder Stift ausgebildet, vorzugsweise mit einem zur Werkzeughaltehülse gerichteten balligen oder kugeligen Ende. Bevorzugt ist ein Fixierelement vorgesehen, es können jedoch auch mehrere Fixierelemente, insbesondere im Wesentlichen gleichmäßig um die Werkzeughaltehülse verteilt, vorgesehen sein.

Vorzugsweise ist das Fixierelement in einer sich radial zur Mittelachse der Werkzeughaltehülse erstreckenden Führung aufgenommen, zum Beispiel in einer Nut oder einer Bohrung. Die Führung lagert und sichert das Fixierelement im Handstück und gewährleistet eine zuverlässige radiale Bewegung des Fixierelements zu der Werkzeughaltehülse und von der Werkzeughaltehülse weg. Die Führung und insbesondere das Fixierelement sind vorzugsweise in dem Kontaktbereich des Fixierelements mit der Werkzeughaltehülse vorgesehen, insbesondere in jenem axialen Abschnitt der Werkzeughaltehülse, welcher die Ausnehmung zur Aufnahme des Fixierelements aufweist.

Vorzugsweise ist das Fixierelement zwischen einer die Werkzeughaltehülse kontaktierenden Position und einer die Werkzeughaltehülse nicht kontaktierenden Position bewegbar. In der kontaktierenden Position ist das Fixierelement radial an die Mittelachse der Werkzeughaltehülse angenähert. In der kontaktierenden Position greift das Fixierelement insbesondere in die zumindest eine, vorzugsweise konkav geformte, Ausnehmung ein, so dass die Werkzeughaltehülse in einer festgelegten oder vorbestimmten Drehposition fixiert ist. In der nicht kontaktierenden Position ist das Fixierelement radial von der Mittelachse der Werkzeughaltehülse weiter entfernt als in der kontaktierenden Position. In der nicht kontaktierenden Position greift das Fixierelement insbesondere nicht in die zumindest eine, vorzugsweise konkav geformte, Ausnehmung ein, so dass die Werkzeughaltehülse frei um ihre Mittelachse drehbar ist, wenn auf die Werkzeughaltehülse und / oder ein darin aufgenommenes Werkzeug ein Drehmoment ausgeübt wird.

Gemäß einem Ausführungsbeispiel weist das Stellelement ein längliches, vorzugsweise stabförmiges, Element auf. Vorzugsweise ist das Stellelement als Stift ausgebildet. Das Stellelement weist des Weiteren eine Längsachse auf, die insbesondere im Wesentlichen parallel zur Mittelachse der Werkzeughaltehülse angeordnet ist.

Vorzugsweise ist das Stellelement derart im Handstück gelagert oder angeordnet, dass es im Wesentlichen entlang seiner Längsachse bewegbar, insbesondere verschiebbar, ist. Vorzugsweise ist das Stellelement in einer Führung aufgenommen, zum Beispiel in einer Nut oder einer Bohrung. Die Führung gewährleistet eine zuverlässige Bewegung des Stellelements entlang seiner Längsachse. Besonders bevorzugt ist ein gemeinsames Führungselement vorgesehen, in dem das Fixierelement und das Stellelement gemeinsam angeordnet und / oder aufgenommen und / oder geführt sind. Vorzugsweise sind die Bohrung oder Nut zur Führung des Stellelements und die Bohrung oder Nut zur Führung des Fixierelements in etwa rechtwinkelig zueinander angeordnet, insbesondere in dem gemeinsamen Führungselement. Vorzugsweise ist die Führung oder das gemeinsame Führungselement fest im Handstück angeordnet und sind das Stellelement und das Fixierelement relativ zum Führungselement beweglich angeordnet.

Gemäß einem Ausführungsbeispiel weist das Stellelement entlang seiner Längsachse, insbesondere in einem Kontaktbereich mit dem Fixierelement, unterschiedliche Außendurchmesser auf. Die Abschnitte des Stellelements mit unterschiedlichen Außendurchmessern sind zum Beispiel durch eine Stufe oder eine Schulter am Stellelement oder durch einen schräg oder gewinkelt zur Längsachse ausgebildeten Verbindungsabschnitt des Stellelements miteinander verbunden. Alternativ weist das Stellelement über zumindest einen Teil seiner Länge einen sich stetig verändernden Außendurchmesser auf.

Das Bewegen des Stellelements entlang seiner Längsachse bewirkt oder ermöglicht aufgrund der operativen oder unmittelbaren, direkten Verbindung des Stellelements mit dem Fixierelement die im Wesentlichen radiale Bewegung des Fixierelements zur oder von der Werkzeughaltehülse. Vorzugsweise ist das Stellelement derart im Handstück angeordnet, dass durch das Bewegen des Stellelements, vorzugsweise entlang seiner Längsachse, wahlweise ein erster Abschnitt des Stellelements mit einem ersten, zum Beispiel großen, Außendurchmesser oder ein zweiter Abschnitt des Stellelements mit einem zweiten, zum Beispiel geringen, Außendurchmesser dem Fixierelement gegenüber steht oder operativ zugeordnet ist. Vorzugsweise ist der erste Außendurchmesser derart bemessen, dass, wenn der erste Außendurchmesser dem Fixierelement gegenüber steht oder operativ zugeordnet ist, das Fixierelement in die, vorzugsweise konkav geformte, Ausnehmung der Werkzeughaltehülse eingreift. Insbesondere ist der erste Außendurchmesser derart bemessen, dass der erste Abschnitt mit dem ersten Außendurchmesser das Fixierelement in die Ausnehmung bewegt oder drückt oder das Fixierelement zwischen dem ersten Abschnitt und der Werkzeughaltehülse, insbesondere der Ausnehmung, geklemmt ist. Vorzugsweise ist der zweite Außendurchmesser derart bemessen, dass, wenn der zweite Außendurchmesser dem Fixierelement gegenüber steht oder operativ zugeordnet ist, das Fixierelement nicht in die, vorzugsweise konkav geformte, Ausnehmung der Werkzeughaltehülse eingreift oder aus der Ausnehmung entfernbar ist oder in seiner Führung bewegbar ist.

Gemäß einem Ausführungsbeispiel weist das Handstück ein das Stellelement vorspannendes Federelement, vorzugsweise eine Spiralfeder, auf. Vorzugsweise ist an dem Stellelement ein Vorsprung oder Flansch vorgesehen, an dem das Federelement lagert. Das Federelement, vorzugsweise auch ein Teil des Stellelements, ist /sind insbesondere in einer Aufnahme oder Bohrung des Handstücks, zum Beispiel in einer Aufnahme oder Bohrung der Außenhülse, angeordnet. Das Federelement spannt das Stellelement in eine erste Position vor, in welcher zum Beispiel der erste Abschnitt des Stellelements mit dem ersten Außendurchmesser dem Fixierelement gegenüber steht, so dass das Fixierelement in die, vorzugsweise konkav geformte, Ausnehmung der Werkzeughaltehülse eingreift. Das Federelement ist des Weiteren derart ausgebildet, dass durch das Ausüben einer der Federkraft des Federelements entgegenwirkenden Kraft, beispielsweise mittels eines Betätigungselements, das Federelement in seiner Form veränderbar, zum Beispiel komprimierbar, ist, wodurch das Stellelement in eine zweite Position bewegbar ist, in welcher der Abschnitt des Stellelements mit dem zweiten Außendurchmesser dem Fixierelement gegenüber steht und das Fixierelement nicht in die Ausnehmung der Werkzeughaltehülse eingreift oder aus der Ausnehmung entfernbar ist. Selbstverständlich ist es auch möglich, das Federelement und / oder das Stellelement derart auszubilden oder anzuordnen, dass das Federelement das Stellelement in die zweite Position vorspannt und durch das Ausüben einer der Federkraft des Federelements entgegenwirkenden Kraft das Stellelement in die erste Position bewegbar ist.

Gemäß einem Ausführungsbeispiel umfasst das Handstück ein relativ zur Außenhülse des Handstücks bewegbares, insbesondere drehbares oder verschwenkbares, Betätigungselement zum Betätigen der Stellvorrichtung, insbesondere des Stellelements. Vorzugsweise ist das Betätigungselement operativ mit dem Stellelement verbunden, insbesondere ist das Betätigungselement ausgebildet, das Stellelement im Wesentlichen parallel zur Mittelachse der Werkzeughaltehülse oder entlang seiner Längsachse zu bewegen. Das Betätigungselement ist zum Beispiel als Kappe oder Taster ausgebildet. Das Betätigungselement ist vorzugsweise als Teil der Außenhülse des Handstücks ausgebildet und / oder es ist in einer Öffnung der Außenhülse aufgenommen.

Das Betätigungselement ist bewegbar, insbesondere drehbar oder verschwenkbar, an der Außenhülse gelagert und / oder mit der Außenhülse verbunden und / oder an mit der Außenhülse, insbesondere drehfest oder unbeweglich, verbundenen Bauteilen des Handstücks gelagert und / oder mit diesen Bauteilen verbunden. Die mit der Außenhülse verbundenen Bauteile umfassen zum Beispiel Stifte, welche eine Dreh- oder Schwenkachse für das Betätigungselement bilden, und / oder Lagerelemente, insbesondere Kugeln, auf denen das Betätigungselement bewegbar gelagert ist, und / oder vorzugsweise die Führung für das Stellelement oder das Fixierelement oder das gemeinsame Führungselement des Stell- und Fixierelements. Vorzugsweise sind auf dem gemeinsamen Führungselement die Lagerelemente, auf denen das Betätigungselement bewegbar gelagert ist, angeordnet.

Gemäß einem Ausführungsbeispiel umfasst das Handstück eine Verriegelungsvorrichtung, die ausgebildet ist, das Betätigungselement oder das Fixierelement zumindest in der im Vorstehenden beschriebenen ersten Position, in welcher das Fixierelement in die Ausnehmung der Werkzeughaltehülse eingreift und die Werkzeughaltehülse in einer Drehposition fixiert, oder in der im Vorstehenden beschriebenen zweiten Position, in welcher das Fixierelement nicht in die Ausnehmung der Werkzeughaltehülse eingreift und das Fixierelement eine freie Drehbarkeit der Werkzeughaltehülse zulässt, zu verriegeln. Insbesondere ist die Verriegelungsvorrichtung derart ausgebildet, dass sie das Betätigungselement in der ersten Position und / oder in der zweiten Position hält oder lagert, damit das Fixierelement in die Ausnehmung der Werkzeughaltehülse eingreift oder nicht eingreift. Vorzugsweise umfasst die Verriegelungsvorrichtung ein erstes Verriegelungsteil an dem Betätigungselement und ein zweites Verriegelungsteil, wobei durch die Bewegung des Betätigungselements das erste Verriegelungsteil und das zweite Verriegelungsteil relativ zueinander bewegbar sind. Insbesondere ist das zweite Verriegelungsteil an einem unbeweglich in dem Handstück oder in der Außenhülse angeordneten Bauteil vorgesehen, vorzugsweise an dem gemeinsamen Führungselement des Stell- und Fixierelements.

Die Verriegelungsvorrichtung, insbesondere das erste Verriegelungsteil oder das zweite Verriegelungsteil, umfasst / umfassen zum Beispiel einen Fortsatz oder Vorsprung, der in einer Aufnahme aufnehmbar oder daraus entfernbar ist. Die Aufnahme umfasst vorzugsweise zumindest ein federndes oder elastisch biegsames Bauteil, zum Beispiel einen Federarm, der vorzugsweise mit einer Nase zum Halten des Fortsatzes versehen ist. Die Aufnahme, insbesondere der zumindest eine Federarm, umfasst zum Beispiel des Weiteren eine Lagerfläche zum Stützen oder Lagern des Vorsprungs, wobei die Lagerfläche bevorzugt an einem freien Ende der Aufnahme oder des Federarms vorgesehen ist. Bevorzugt ist der Fortsatz an dem Betätigungselement vorgesehen. Bevorzugt ist die Aufnahme an einem unbeweglich in dem Handstück oder in der Außenhülse angeordneten Bauteil vorgesehen, vorzugsweise an dem gemeinsamen Führungselement des Stell- und Fixierelements.

Gemäß einem Ausführungsbeispiel sind die Werkzeughaltehülse und die Antriebswelle durch ein Exzentergetriebe miteinander verbunden. Bevorzugt umfasst das Exzentergetriebe einen Exzenterstift, der insbesondere an der Antriebswelle des Handstücks vorgesehen ist, und eine Nut zur Aufnahme des Exzenterstifts, die insbesondere an der Außenfläche oder am Außenmantel der Werkzeughaltehülse vorgesehen ist.

Gemäß einem Ausführungsbeispiel weist das Handstück einen Handstückkopf auf, in welchem die Werkzeughaltehülse derart angeordnet ist, dass die Mittelachse der Werkzeughaltehülse gewinkelt zu einer Drehachse der Antriebswelle verläuft, wobei des Weiteren am Handstückkopf das Fixierelement, das Stellelement, das Betätigungselement und zumindest Teile der Verriegelungsvorrichtung vorgesehen sind. Insbesondere ist in dem Handstückkopf das gemeinsame Führungselement vorgesehen, welches zumindest zur Führung des Stell- und Fixierelements, vorzugsweise auch zur bewegbaren Lagerung des Betätigungselements und / oder zur Lagerung zumindest eines Teils der Verriegelungsvorrichtung ausgebildet ist.

Die Erfindung wird nachfolgend anhand bevorzugter Ausführungsbeispiele und Bezug nehmend auf die beigefügten Zeichnungen erläutert. Es zeigt die
Figur 1 ein medizinisches, insbesondere dentales, Handstück mit einer in eine Hubbewegung versetzbaren Werkzeughaltehülse und einer Stellvorrichtung, die ausgebildet ist, die Werkzeughaltehülse wahlweise in einer Drehposition zu fixieren oder eine freie Drehbarkeit der Werkzeughaltehülse zuzulassen.
Figur 2 eine vergrößerte Darstellung des Handstückkopfs des Handstücks der Figur 1, wobei die Stellvorrichtung eine erste Position einnimmt, in welcher die Werkzeughaltehülse in einer Drehposition fixiert ist.
Figur 3 eine vergrößerte Darstellung des Handstückkopfs des Handstücks der Figur 1, wobei die Stellvorrichtung eine zweite Position einnimmt, in welcher die Werkzeughaltehülse frei drehbar ist.
Figur 4 eine weitere Schnittdarstellung durch den Handstückkopf des Handstücks der Figur 1, wobei die Schnittebene in Bezug auf die Figuren 2 und 3 um 90° gedreht ist.
Figur 5 den Handstückkopf des Handstücks der Figur 1 in Aufsicht mit einem Betätigungselement für die Stellvorrichtung und einer Verriegelungsvorrichtung für das Betätigungselement und / oder die Stellvorrichtung.

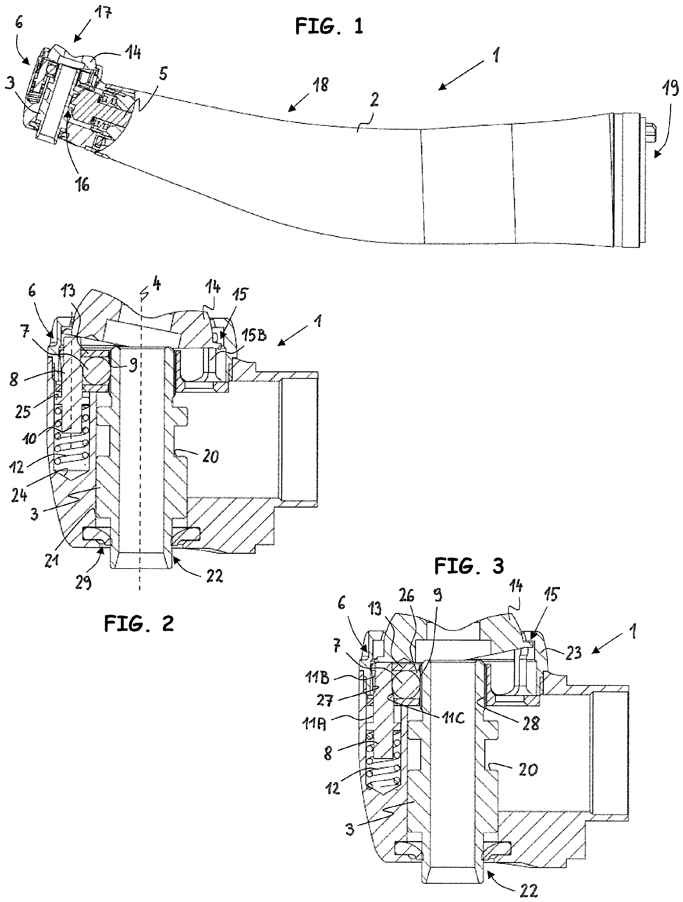

Das medizinische, insbesondere dentale, Handstück 1 der Figuren 1 - 5 umfasst einen Handstückkopf 17 und einen damit verbundenen, vorzugsweise gebogenen oder gewinkelten, Griffteil 18. Eine Außenhülse 2 umgibt den Handstückkopf 17 und das Griffteil 18. An einem Ende des Handstücks 1 ist eine Verbindungsvorrichtung 19 vorgesehen, die ausgebildet ist, das Handstück 1 mit einer Antriebsvorrichtung zu verbinden, zum Beispiel mit einem Motor, insbesondere einem Elektromotor. Eine oder mehrere Antriebswellen 5 erstrecken sich von der Verbindungsvorrichtung 19 durch das Griffteil 18 in Richtung des Handstückkopfs 17. Vorzugsweise ist die Verbindungsvorrichtung 19 auch zur Verbindung des Handstücks 1 mit einer Energie-, Licht- oder Fluidquelle ausgebildet.

Im Handstückkopf 17 ist eine Werkzeughaltehülse 3 vorgesehen, die zur lösbaren Aufnahme eines Behandlungswerkzeugs, insbesondere eines Werkzeugs mit einem flächigen Arbeitsabschnitt, zum Beispiel einer Feile, ausgebildet ist. Die Werkzeughaltehülse 3 ist mit der zumindest einen Antriebswelle 5 über ein Exzentergetriebe 16 verbunden, welches ausgebildet ist, die von der Antriebswelle 5 übertragene Drehbewegung derart auf die Werkzeughaltehülse 3 zu übertragen, dass die Werkzeughaltehülse 3 in eine Hubbewegung versetzbar ist. Das Exzentergetriebe 16 umfasst einen an der Antriebswelle 5 vorgesehenen Exzenterstift und eine die Außenfläche der Werkzeughaltehülse 3 ringförmig umgebende Nut 20 zum Eingriff des Exzenterstifts. Die Werkzeughaltehülse 3 ist mittels zumindest eines Gleitlagers 21 in dem Handstück 1 gelagert.

Am Handstückkopf 17 ist eine Werkzeugaufnahmeöffnung 22 vorgesehen, durch welche das Behandlungswerkzeug in den Handstückkopf 17 oder die Werkzeughaltehülse 3 einsetzbar oder daraus entnehmbar ist. In der Werkzeugaufnahmeöffnung 22 oder anschließend an die Werkzeugaufnahmeöffnung 22 ist die Werkzeughaltehülse 3 derart im Handstückkopf 17 angeordnet, dass sich ihre Mittelachse 4 gewinkelt zu einer Drehachse der Antriebswelle 5 erstreckt. Die Werkzeugaufnahmeöffnung 22 ist somit an einer Seite des Handstückkopfs 17 vorgesehen. An der Werkzeugaufnahmeöffnung 22 oder um die Werkzeughaltehülse 3 ist eine Dichtvorrichtung 29 vorgesehen, zum Beispiel eine elastischer Dichtring, die das Eindringen von Verunreinigungen in das Handstück 1 verhindert.

Die Werkzeughaltehülse 3 ist des Weiteren drehbar um eine Dreh- oder Mittelachse 4 der Werkzeughaltehülse 3 in dem Handstück 1 gelagert, so dass ein in der Werkzeughaltehülse 3 aufgenommenes Behandlungswerkzeug in unterschiedlichen Drehwinkeln oder Drehpositionen anordenbar ist. Zur wahlweisen Fixierung der Werkzeughaltehülse 3 oder des darin aufgenommenen Behandlungswerkzeugs in einer bestimmten Drehposition oder alternativ zum freien Drehen der Werkzeughaltehülse 3 oder des darin aufgenommenen Behandlungswerkzeugs ist am Handstück 1 eine Stellvorrichtung 6 vorgesehen. Zur Betätigung der Stellvorrichtung 6 ist am Handstückkopf 17 ein Betätigungselement 14 vorgesehen, das insbesondere auf der Seite des Handstückkopfs 17 vorgesehen ist, die der Seite des Handstückkopfs 17 mit der Werkzeugaufnahmeöffnung 22 gegenüber liegt.

Das Betätigungselement 14 umfasst einen Taster oder Druckknopf, der verschwenkbar auf dem Handstückkopf 17 angeordnet ist. Das Betätigungselement 14 ragt insbesondere durch eine Öffnung einer Kappe 23, die mit der Außenhülse 2 lösbar verbunden ist, zum Beispiel mittels eines Gewindes, und / oder als Teil der Außenhülse 2 ausgebildet ist.

Die Stellvorrichtung 6 umfasst ein Stellelement 8 in Form eines länglichen, vorzugsweise zylindrischen, Stifts, der sich entlang einer Längsachse 10 erstreckt, und ein Fixierelement 7 in Form einer Kugel. Das Stellelement 8 weist einen ersten Abschnitt mit einem ersten, großen Außendurchmesser 11A, einen zweiten Abschnitt mit einem zweiten, geringen Außendurchmesser 11B und einen schräg zur Längsachse 10 des Stellelements 8 ausgebildeten Verbindungsabschnitt 11C auf, der den ersten und den zweiten Abschnitt verbindet (siehe insbesondere Figur 3). Des Weiteren ist das Stellelement 8 durch ein Federelement 12 in Form einer Spiralfeder vorgespannt. Das Federelement 12 ist in einer Bohrung 24 des Handstücks 1, insbesondere der Außenhülse 2, aufgenommen und an einem Flansch 25 des Stellelements 8 gelagert (siehe Figur 2).

Wie aus einem Vergleich der beiden Figuren 2 und 3 erkennbar ist, ist das Stellelement 8 entlang seiner Längsachse 10, im Wesentlichen parallel zur Mittelachse 4 der Werkzeughaltehülse 3 derart verschiebbar, dass entweder der erste, große Außendurchmesser 11A oder der zweite, geringe Außendurchmesser 11B dem Fixierelement 7 gegenüber stehen oder operativ zugeordnet sind. Letzteres erfolgt, wenn das Federelement 12 komprimiert ist und das Stellelement 8 entgegen der Federkraft des Federelements 12 in Richtung der Werkzeugaufnahmeöffnung 22 verschoben ist. Durch die selbsttätige Expansion des Federelements 12 aus der komprimierten Position wird das Stellelement 8 von der Werkzeugaufnahmeöffnung 22 weg bewegt.

Steht der erste, große Außendurchmesser 11A dem Fixierelement 7 gegenüber, dann kontaktiert das Stellelement 8, insbesondere der erste Abschnitt mit einem ersten, großen Außendurchmesser 11A, das Fixierelement 7, insbesondere derart, dass das Fixierelement 7 in eine rillen- oder nutförmige, vorzugsweise konkave, Ausnehmung 9 an der Außenseite der Werkzeughaltehülse 3 eingreift oder dass das Fixierelement 7 zwischen der Werkzeughaltehülse 3 und dem Stellelement 8 geklemmt ist (siehe Figur 2). Damit ist die Werkzeughaltehülse 3 und ein darin aufgenommenes Werkzeug in einer Drehposition fixiert. Steht der zweite, geringe Außendurchmesser 11B dem Fixierelement 7 gegenüber, dann erhält das Fixierelement 7 Platz, sich von der Werkzeughaltehülse 3 weg oder in Richtung des Stellelements 8 zubewegen, insbesondere derart, dass das Fixierelement 7 nicht in die rillen- oder nutförmige Ausnehmung 9 an der Außenseite der Werkzeughaltehülse 3 eingreift oder dass das Fixierelement 7 nicht zwischen der Werkzeughaltehülse 3 und dem Stellelement 8 geklemmt ist (siehe Figur 3). Damit ist die Werkzeughaltehülse 3 und ein darin aufgenommenes Werkzeug frei drehbar. Die Ausnehmung 9 ist vorzugsweise in die Außenseite oder in den Außenmantel der Werkzeughaltehülse 3 geformt.

Gemäß einem alternativen, nicht dargestellten Ausführungsbeispiel ist das Fixierelement 7 durch einen am Stellelement 8 vorgesehenen und mit dem Stellelement 8 im Wesentlichen parallel zur Mittelachse 4 der Werkzeughaltehülse 3 bewegbaren Abschnitt gebildet. Insbesondere ist dieser Abschnitt durch einen ersten Abschnitt des Stellelements 8 gebildet, der einen größeren Durchmesser aufweist, als ein zweiter Abschnitt des Stellelements 8. Vorzugsweise umfasst dieser erste Abschnitt den ersten Abschnitt mit einem ersten, großen Außendurchmesser 11A und der zweite Abschnitt den zweiten Abschnitt mit einem zweiten, geringen Außendurchmesser 11B. Vorzugsweise ist dieser Abschnitt mit dem größeren Durchmesser (zum Beispiel der erste Abschnitt 11A) ausgebildet, wahlweise in die zumindest eine, vorzugsweise konkav geformte, Ausnehmung 9 an der Außenfläche der Werkzeughaltehülse 3 einzugreifen. Alternativ ist dieser Abschnitt mit dem größeren Durchmesser (zum Beispiel der erste Abschnitt 11A) ausgebildet, wahlweise in eine, vorzugsweise konkav geformte, Ausnehmung zur Aufnahme des Fixierelements 7 eines Bauteils einzugreifen, welches drehfest mit der Werkzeughaltehülse 3 verbunden ist. Das Bauteil ist zum Beispiel als Scheibe ausgebildet, die an ihrem Außenumfang zumindest eine Ausnehmung zur Aufnahme des Fixierelements 7 aufweist und an der eine, zum Beispiel polygone, Bohrung vorgesehen ist, in welcher die Werkzeughülse 3 aufnehmbar oder aufgenommen ist. Im Übrigen erfolgt das wahlweise Fixieren oder freie Drehen der Werkzeughülse 3 und eine darin aufgenommenen Werkzeugs entsprechend wie im Vorstehenden beschrieben, d.h. durch Verschieben des Stellelements 8 mit dem Fixierelement 7, 11A, so dass das Fixierelement 7, 11A wahlweise in die Ausnehmung zur Aufnahme des Fixierelements 7 eingreift oder nicht eingreift.

Das Fixierelement 7 und das Stellelement 8 sind insbesondere in einem gemeinsamen Führungselement 13 angeordnet. Das gemeinsame Führungselement 13 weist eine erste Bohrung 26 (siehe Figur 3) auf, in der das Fixierelement 7 beweglich gelagert ist, insbesondere radial beweglich zur Mittelachse 4 der Werkzeughaltehülse 3. Das gemeinsame Führungselement 13 weist eine zweite Bohrung 27 auf, in der das Stellelement 8 beweglich, insbesondere entlang seiner Längsachse 10 und im Wesentlichen parallel zur Mittelachse 4, aufgenommen ist. Die Bohrungen 26, 27 sind im Wesentlichen rechtwinkelig zueinander angeordnet. Ein dritte Bohrung 28 in dem gemeinsamen Führungselement 13 ist zur Aufnahme zumindest eines Abschnitts der Werkzeughaltehülse 3 ausgebildet, insbesondere zur Aufnahme des Abschnitts der Werkzeughaltehülse 3 mit den Ausnehmungen 9. Die drei Bohrungen 26, 27, 28 sind miteinander verbunden. Das gemeinsame Führungselement 13 ist drehfest in dem Handstück 1 oder dem Handstückkopf 17, insbesondere an der Außenhülse 2, befestigt.

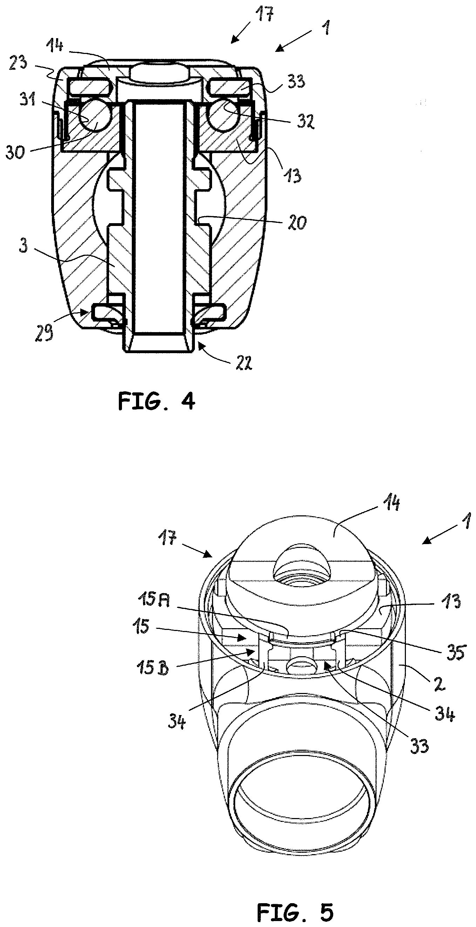

Das beweglich im Handstück 1 angeordnete Betätigungselement 14 ist durch zumindest ein Lagerelement 30 beweglich, insbesondere verschwenkbar, im Handstück 1 gelagert. Das Lagerelement 30 umfasst zum Beispiel eine oder mehrere Kugeln, die in Aufnahmen 31 in dem Handstück 1 angeordnet sind. Die Aufnahmen 31 sind zum Beispiel in dem gemeinsamen Führungselement 13 vorgesehen und insbesondere derart bemessen, dass ein Teil des Lagerelements 30 aus der Aufnahme 31 ragt und das Betätigungselement 14 lagert (siehe Figur 4). Vorzugsweise sind in dem Betätigungselement 14 Aufnahmen oder Rücksprünge 32 vorgesehen, in welche der über das gemeinsame Führungselement 13 ragende Teil des Lagerelements 30 eingreift. Des Weiteren sind am Betätigungselement 14 zwei Stifte 33 vorgesehen, die als Dreh- oder Schwenkachse für das Betätigungselement 14 vorgesehen sind. Die Stellvorrichtung 6, insbesondere das Stellelement 8, ist operativ und / oder direkt mit dem Betätigungselement 14 verbunden. Eine Bewegung des Betätigungselements 14 ist somit auf die Stellvorrichtung 6, insbesondere das Stellelement 8, übertragbar, wodurch das Stellelement 8 entlang seiner Längsachse 10 verschiebbar ist und, wie im Vorstehenden beschrieben, die Werkzeughaltehülse 3 und ein darin aufgenommenes Werkzeug entweder in einer Drehposition fixiert oder frei drehbar ist.

Eine am Handstück 1 vorgesehene Verriegelungsvorrichtung 15 ist ausgebildet, das Betätigungselement 14 oder das Fixierelement 7 in einer ersten Position, in welcher das Fixierelement 7 die Werkzeughaltehülse 3 in einer Drehposition fixiert, und / oder in einer zweiten Position, in welcher das Fixierelement 7 eine freie Drehbarkeit der Werkzeughaltehülse 3 zulässt, zu verriegeln. Die Verriegelungsvorrichtung 15 umfasst ein erstes Verriegelungsteil 15A und ein zweites Verriegelungsteil 15B (siehe insbesondere Figur 5). Das erste Verriegelungsteil 15A ist am Betätigungselement 14 vorgesehen und umfasst einen Fortsatz oder Vorsprung. Das zweite Verriegelungsteil 15B ist am gemeinsamen Führungselement 13 vorgesehen und umfasst eine Aufnahme 33, in welcher das erste Verriegelungsteil 15A aufnehmbar ist. Die Aufnahme 33 ist durch zwei federnde oder elastisch biegsame Federarme 34 gebildet oder begrenzt, die vorzugsweise mit jeweils einer Nase zum Halten des ersten Verriegelungsteils 15A versehen sind (siehe auch Figur 2). Die Aufnahme 33, insbesondere die Federarme 34, sind auf dem gemeinsamen Führungselement 13 vorgesehen oder daran befestigt. Die Aufnahme 33, insbesondere die Federarme 34, umfasst / umfassen des Weiteren eine Lagerfläche 35 zum Stützen oder Lagern des ersten Verriegelungsteils 15A (siehe Figur 5).

Die Verriegelungsvorrichtung 15 ist somit ausgebildet, das Betätigungselement 14 und somit die Stellvorrichtung 6 in zwei Positionen zu halten oder zu verriegeln, die den im Vorstehenden beschriebenen und in den Figuren 2 und 3 dargestellten Positionen entsprechen: In einer Position steht der erste, große Außendurchmesser 11A des Stellelements 8 dem Fixierelement 7 gegenüber oder ist dem Fixierelement 7 operativ zugeordnet, so dass die Werkzeughaltehülse 3 und ein darin aufgenommenes Werkzeug in einer Drehposition fixiert (siehe Figur 2); in der anderen Position steht der zweite, geringe Außendurchmesser 11B des Stellelements 8 dem Fixierelement 7 gegenüber oder ist dem Fixierelement 7 operativ zugeordnet, so dass die Werkzeughaltehülse 3 und ein darin aufgenommenes Werkzeug frei drehbar sind (siehe Figur 3).

Die Erfindung ist nicht auf die beschriebenen Ausführungsbeispiele beschränkt, sondern umfasst alle Ausführungen, die dem durch die Ansprüche definierten Schutzbegehren entsprechen. Des Weiteren sind alle Merkmale aller beschriebenen und dargestellten Ausführungsbeispiele miteinander kombinierbar.

## Patentansprüche

1. Medizinisches, insbesondere dentales, Handstück (1), umfassend: Eine Außenhülse (2), eine Werkzeughaltehülse (3) zum Halten eine Behandlungswerkzeugs, wobei die Werkzeughaltehülse (3) eine Mittelachse (4) aufweist, um welche die Werkzeughaltehülse (3) drehbar ist, und wobei die Werkzeughaltehülse (3) durch ein Antriebswelle (5) des Handstücks (1) in eine Hubbewegung versetzbar ist, und eine Stellvorrichtung (6), die ausgebildet ist, die Werkzeughaltehülse (3) wahlweise in einer Drehposition zu fixieren oder eine freie Drehbarkeit der Werkzeughaltehülse (3) zuzulassen, wobei die Stellvorrichtung (6) ein die Werkzeughaltehülse (3) fixierendes oder freigebendes Fixierelement (7) und ein zumindest operativ mit dem Fixierelement (7) verbundenes Stellelement (8) aufweist, wobei das Stellelement (8) im Wesentlichen parallel zur Mittelachse (4) der Werkzeughaltehülse (3) bewegbar ist, **dadurch gekennzeichnet, dass**
die Werkzeughaltehülse (3) an ihrer Außenfläche zumindest eine, vorzugsweise konkav geformte, Ausnehmung (9) zur Aufnahme des Fixierelements (7) aufweist.

2. Medizinisches, insbesondere dentales, Handstück (1) nach Anspruch 1, **dadurch gekennzeichnet, dass**
das Fixierelement (7) zumindest ein relativ zur Mittelachse (4) der Werkzeughaltehülse (3) im Wesentlichen radial bewegbares, vorzugsweise kugelförmiges oder ein balliges Ende aufweisendes, Formelement umfasst.

3. Medizinisches, insbesondere dentales, Handstück (1) nach Anspruch 1 oder 2, **dadurch gekennzeichnet, dass**
das Fixierelement (7) zwischen einer die Werkzeughaltehülse (3) kontaktierenden Position und einer die Werkzeughaltehülse (3) nicht kontaktierenden Position bewegbar ist.

4. Medizinisches, insbesondere dentales, Handstück (1) nach Anspruch 1, **dadurch gekennzeichnet, dass**
das Fixierelement (7) einen am Stellelement (8) vorgesehenen und mit dem Stellelement (8) im Wesentlichen parallel zur Mittelachse (4) der Werkzeughaltehülse (3) bewegbaren Abschnitt (11A) aufweist.

5. Medizinisches, insbesondere dentales, Handstück (1) nach einem der vorstehenden Ansprüche, **dadurch gekennzeichnet, dass**
die zumindest eine Ausnehmung (9) als längliche Rille oder Nut ausgebildet ist, die sich entlang der Außenfläche der Werkzeughaltehülse (3), insbesondere im Wesentlichen parallel zur Mittelachse (4) der Außenhülse (2), erstrecken.

6. Medizinisches, insbesondere dentales, Handstück (1) nach einem der vorstehenden Ansprüche, **dadurch gekennzeichnet, dass**
das Stellelement (8) ein längliches, vorzugsweise stabförmiges, Element aufweist, dessen Längsachse (10) im Wesentlichen parallel zur Mittelachse (4) der Werkzeughaltehülse (3) angeordnet ist.

7. Medizinisches, insbesondere dentales, Handstück (1) nach Anspruch 6, **dadurch gekennzeichnet, dass**
das Stellelement (8) im Wesentlichen entlang seiner Längsachse (10) bewegbar ist.

8. Medizinisches, insbesondere dentales, Handstück (1) nach einem der vorstehenden Ansprüche, **dadurch gekennzeichnet, dass**
das Stellelement (8) entlang seiner Längsachse (10), insbesondere in einem Kontaktbereich mit dem Fixierelement (7), unterschiedliche Außendurchmesser (11A, 11B)aufweist.

9. Medizinisches, insbesondere dentales, Handstück (1) nach Anspruch 8, **dadurch gekennzeichnet, dass**
zwei Abschnitte des Stellelements (8), welche unterschiedliche Außendurchmesser (11A, 11B) aufweisen, durch einen schräg zur Längsachse (10) des Stellelements (8) ausgebildeten Verbindungsabschnitt (11C) des Stellelements (8) miteinander verbunden sind.

10. Medizinisches, insbesondere dentales, Handstück (1) nach einem der vorstehenden Ansprüche, **gekennzeichnet durch**
ein das Stellelement (8) vorspannendes Federelement (12), vorzugsweise eine Spiralfeder.

11. Medizinisches, insbesondere dentales, Handstück (1) nach einem der vorstehenden Ansprüche, **gekennzeichnet durch**
ein in der Außenhülse (2) angeordnetes gemeinsames Führungselement (13), welches zur Führung des Stellelements (8) und des Fixierelements (7) ausgebildet ist.

12. Medizinisches, insbesondere dentales, Handstück (1) nach einem der vorstehenden Ansprüche, **gekennzeichnet durch**
ein relativ zur Außenhülse (2) des Handstücks (1) bewegbares, insbesondere drehbares oder verschwenkbares, Betätigungselement (14) zum Betätigen der Stellvorrichtung (6).

13. Medizinisches, insbesondere dentales, Handstück (1) nach einem der vorstehenden Ansprüche, **gekennzeichnet durch**
eine Verriegelungsvorrichtung (15), die ausgebildet ist, das Betätigungselement (14) oder das Fixierelement (7) zumindest in einer ersten Position, in welcher das Fixierelement (7) die Werkzeughaltehülse (3) in einer Drehposition fixiert, oder in einer zweiten Position, in welcher das Fixierelement (7) eine freie Drehbarkeit der Werkzeughaltehülse (3) zulässt, zu verriegeln.

14. Medizinisches, insbesondere dentales, Handstück (1) nach Anspruch 13, **dadurch gekennzeichnet, dass**
die Verriegelungsvorrichtung (15) ein erstes Verriegelungsteil (15A) an dem Betätigungselement (14) und ein zweites Verriegelungsteil (15B), insbesondere an dem gemeinsamen Führungselement (13), umfasst, wobei durch die Bewegung des Betätigungselements (14) das erste Verriegelungsteil (15A) und das zweite Verriegelungsteil (15B) relativ zueinander bewegbar sind.

15. Medizinisches, insbesondere dentales, Handstück (1) nach einem der vorstehenden Ansprüche, **dadurch gekennzeichnet, dass**
das Handstück (1) einen Handstückkopf (17) aufweist, in welchem die Werkzeughaltehülse (3) derart angeordnet ist, dass die Mittelachse (4) der Werkzeughaltehülse (3) gewinkelt zu einer Drehachse der Antriebswelle (5) verläuft, wobei des Weiteren am Handstückkopf (17) das Fixierelement (7), das Stellelement (8) und das Betätigungselement vorgesehen sind.

## Claims

1. A medical, in particular dental, handpiece (1), comprising: an outer sleeve (2), a tool-holding sleeve (3) for holding a treatment tool, wherein the tool-holding sleeve (3) comprises a central axis (4) about which the tool-holding sleeve (3) can be rotated and wherein the tool-holding sleeve (3) can be induced to a stroke motion by a driveshaft (5) of the handpiece (1), and an adjusting device (6) which is configured to selectively secure the tool-holding sleeve (3) in a rotational position or to allow a free rotatability of the tool-holding sleeve (3), wherein the adjusting device (6) comprises a fixation element (7) which secures or releases the tool-holding sleeve (3) and an adjusting element (8) which is at least operatively connected to the fixation element (7), wherein the adjusting element (8) is movable substantially parallel to the central axis (4) of the tool-holding sleeve (3), **characterized in that**
the tool-holding sleeve (3) comprises on its outside surface at least one recess (9), preferably concave in shape, to receive the fixation element (7).

2. The medical, in particular dental, handpiece (1) according to Claim 1, **characterized in that**
the fixation element (7) comprises at least one form element that is movable substantially radially relative to the central axis (4) of the tool-holding sleeve (3) and preferably is spherical or comprises a convex end.

3. The medical, in particular dental, handpiece (1) according to Claim 1 or 2, **characterized in that**
the fixation element (7) is movable between a position in which it contacts the tool-holding sleeve (3) and a position in which it does not contact the tool-holding sleeve (3).

4. The medical, in particular dental, handpiece (1) according to Claim 1, **characterized in that**
the fixation element (7) comprises a section (11A) which is provided on the adjusting element (8) and is movable together with the adjusting element (8) substantially parallel to the central axis (4) of the tool-holding sleeve (3).

5. The medical, in particular dental, handpiece (1) according to any one of the preceding claims, **characterized in that**
the at least one recess (9) is configured as an elongated groove or channel extending along the outer surface of the tool-holding sleeve (3), in particular substantially parallel to the central axis (4) of the outer sleeve (2).

6. The medical, in particular dental, handpiece (1) according to any one of the preceding claims, **characterized in that**
the adjusting element (8) comprises an elongated, preferably rod-shaped, element whose longitudinal axis (10) is arranged substantially parallel to the central axis (4) of the tool-holding sleeve (3).

7. The medical, in particular dental, handpiece (1) according to Claim 6, **characterized in that**
the adjusting element (8) is movable substantially along its longitudinal axis (10).

8. The medical, in particular dental, handpiece (1) according to any one of the preceding claims, **characterized in that**
the adjusting element (8) comprises different outside diameters (11A, 11B) along its longitudinal axis (10), in particular in a contact area with the fixation element (7).

9. The medical, in particular dental, handpiece (1) according to Claim 8, **characterized in that**
two sections of the adjusting element (8), which have different outside diameters (11A, 11B) are connected to one another by a connecting section (11C) of the adjusting element (8) arranged obliquely to the longitudinal axis (10) of the adjusting element (8).

10. The medical, in particular dental, handpiece (1) according to any one of the preceding claims, **characterized by**
a spring element (12), preferably a spiral spring prestressing the adjusting element (8).

11. The medical, in particular dental, handpiece (1) according to any one of the preceding claims, **characterized by**
a shared guide element (13) arranged in the outer sleeve (2) and configured to guide the adjusting element (8) and the fixation element (7).

12. The medical, in particular dental, handpiece (1) according to any one of the preceding claims, **characterized by**
an actuating element (14) for actuating the adjusting device (6), wherein the actuating element (14) is movable in relation to the outer sleeve (2) of the handpiece (1), in particular being rotatable or pivotable.

13. The medical, in particular dental, handpiece (1) according to any one of the preceding claims, **characterized by**
a locking device (15), which is configured to lock the actuating element (14) or the fixation element (7) at least in a first position, in which the fixation element (7) secures the tool-holding sleeve (3) in a rotational position or in a second position in which the fixation element (7) allows free rotatability of the tool-holding sleeve (3).

14. The medical, in particular dental, handpiece (1) according to Claim 13, **characterized in that**
the locking device (15) comprises a first locking part (15A) on the actuating element (14) and a second locking part (15B), in particular on the shared guide element (13), wherein the first locking part (15A) and the second locking part (15B) are movable in relation to one another by the movement of the actuating element (14).

15. The medical, in particular dental, handpiece (1) according to any one of the preceding claims, **characterized in that**
the handpiece (1) comprises a handpiece head (17) in which the tool-holding sleeve (3) is arranged such that the central axis (4) of the tool-holding sleeve (3) runs at an angle to an axis of rotation of the driveshaft (5) and wherein the fixation element (7), the adjusting element (8) and the actuating element are provided on the handpiece head (17).

## Revendications

1. Pièce à main médicale, en particulier dentaire (1), comprenant: un manchon extérieur (2), un manchon porte-outil (3), destiné à maintenir un outil de traitement, le manchon porte-outil (3) comportant un axe médian (4) autour duquel le manchon porte-outil (3) est rotatif et le manchon porte-outil (3) étant susceptible d'être amené dans une course de levée par un arbre d'entraînement (5) de la pièce à main (1) et un dispositif de réglage (6), qui est conçu, au choix, pour fixer le manchon porte-outil (3) dans une position de rotation ou pour admettre une aptitude libre à la rotation du manchon porte-outil (3), le dispositif de réglage (6) comportant un élément de fixation (7) fixant ou libérant le manchon porte-outil (3) et un élément de réglage (8), assemblé de manière au moins opérationnelle avec l'élément de fixation (7), l'élément de réglage (8) étant mobile sensiblement à la parallèle de l'axe médian (4) du manchon porte-outil (3), **caractérisée en ce que**
sur sa surface extérieure, le manchon porte-outil (3) comporte au moins un évidement (9), façonné de préférence de forme concave, destiné à réceptionner l'élément de fixation (7).

2. Pièce à main médicale, en particulier dentaire (1) selon la revendication 1, **caractérisée en ce que**
l'élément de fixation (7) comprend au moins un élément moulé, de préférence sphérique ou comportant une extrémité bombée, mobile sensiblement en direction radiale par rapport à l'axe médian (4) du manchon porte-outil (3).

3. Pièce à main médicale, en particulier dentaire (1) selon la revendication 1 ou 2, **caractérisée en ce que**
l'élément de fixation (7) est mobile entre une position de contact avec le manchon porte-outil (3) et une position hors contact avec le manchon porte-outil (3).

4. Pièce à main médicale, en particulier dentaire (1) selon la revendication 1, **caractérisée en ce que**
l'élément de fixation (7) comporte un segment (11A) prévu sur l'élément de réglage (8) et mobile avec l'élément de réglage (8) sensiblement à la parallèle de l'axe médian (4) du manchon porte-outil (3).

5. Pièce à main médicale, en particulier dentaire (1) selon l'une quelconque des revendications précédentes, **caractérisée en ce que**
l'au moins un évidement (9) est conçu sous la forme d'une cannelure ou rainure allongée, qui s'étend le long de la surface extérieure du manchon porte-outil (3), en particulier sensiblement à la parallèle de l'axe médian (4) du manchon extérieur (2).

6. Pièce à main médicale, en particulier dentaire (1) selon l'une quelconque des revendications précédentes, **caractérisée en ce que**
l'élément de réglage (8) comporte un élément allongé, de préférence en forme de barre, dont l'axe longitudinal (10) est placé sensiblement à la parallèle de l'axe médian (4) du manchon porte-outil (3).

7. Pièce à main médicale, en particulier dentaire (1) selon la revendication 6, **caractérisée en ce que**
l'élément de réglage (8) est mobile sensiblement le long de son axe longitudinal (10).

8. Pièce à main médicale, en particulier dentaire (1) selon l'une quelconque des revendications précédentes, **caractérisée en ce que**
l'élément de réglage (8) présente différents diamètres extérieurs (11A, 11B) le long de son axe longitudinal (10), en particulier dans une zone de contact avec l'élément de fixation (7).

9. Pièce à main médicale, en particulier dentaire (1) selon la revendication 8, **caractérisée en ce que**
deux segments de l'élément de réglage (8), lesquels comportent différents diamètres extérieurs (11A, 11B) sont assemblés l'un à l'autre par un segment d'assemblage (11C) de l'élément de réglage (8), conçu en oblique de l'axe longitudinal (10) de l'élément de réglage (8).

10. Pièce à main médicale, en particulier dentaire (1) selon l'une quelconque des revendications précédentes, **caractérisée par**
un élément à ressort (12), de préférence un ressort hélicoïdal qui précontraint l'élément de réglage (8).

11. Pièce à main médicale, en particulier dentaire (1) selon l'une quelconque des revendications précédentes, **caractérisée par**
un élément de guidage (13) commun, placé dans le manchon extérieur (2), lequel est conçu pour guider l'élément de réglage (8) et l'élément de fixation (7).

12. Pièce à main médicale, en particulier dentaire (1) selon l'une quelconque des revendications précédentes, **caractérisée par**
un élément de manoeuvre (14) mobile, en particulier rotatif ou pivotant par rapport au manchon extérieur (2) de la pièce à main (1), destiné à manoeuvrer le dispositif de réglage (6).

13. Pièce à main médicale, en particulier dentaire (1) selon l'une quelconque des revendications précédentes, **caractérisée par**
un dispositif de verrouillage (15), qui est conçu pour verrouiller l'élément de manoeuvre (14) ou l'élément de fixation (7) au moins dans une première position, dans laquelle l'élément de fixation (7) fixe le manchon porte-outil (3) dans une position en rotation, ou dans une deuxième position, dans laquelle l'élément de fixation (7) admet une aptitude libre à la rotation du manchon porte-outil (3).

14. Pièce à main médicale, en particulier dentaire (1) selon la revendication 13, **caractérisée en ce que**
le dispositif de verrouillage (15) comprend une première pièce de verrouillage (15A) sur l'élément de manoeuvre (14) et une deuxième pièce de verrouillage (15B), en particulier sur l'élément de guidage (13) commun, par le déplacement de l'élément de manoeuvre (14), la première pièce de verrouillage (15A) et la deuxième pièce de verrouillage (15B) étant mobiles l'une par rapport à l'autre.

15. Pièce à main médicale, en particulier dentaire (1) selon l'une quelconque des revendications précédentes, **caractérisée en ce que**
la pièce à main (1) comporte une tête de pièce à main (17) dans laquelle le manchon porte-outil (3) est placé de telle sorte que l'axe médian (4) du manchon porte-outil (3) s'écoule sous forme coudée par rapport à un axe de rotation de l'arbre d'entraînement (5), sur la tête de pièce à main (17) étant prévus par ailleurs l'élément de fixation (7), l'élément de réglage (8) et l'élément de manoeuvre.
